Europäisches Patentamt

European Patent Office

Office européen des brevets

(19)

(11) Publication number: **0 516 063 A1**

## (12) EUROPEAN PATENT APPLICATION

(21) Application number: **92108876.1**

(22) Date of filing: **26.05.92**

(51) Int. Cl.5: **G01N 33/535**, G01N 33/58

(30) Priority: **29.05.91 JP 155594/91**

(43) Date of publication of application:
**02.12.92 Bulletin 92/49**

(84) Designated Contracting States:
**AT BE CH DE DK ES FR GB GR IT LI LU MC NL PT SE**

(71) Applicant: **SHIONOGI SEIYAKU KABUSHIKI KAISHA trading under the name of SHIONOGI & CO. LTD.**
**1-8, Doshomachi 3-chome Chuo-ku**
**Osaka 541(JP)**

(72) Inventor: **Imagawa, Keiichi**
**5-8-703, Koya-motomachi**
**Neyagawa-shi, Osaka(JP)**
Inventor: **Kosugi, Yoko**
**4-4-3, Takakura-dai**
**Sakai-shi, Osaka(JP)**

(74) Representative: **Vossius & Partner**
**Siebertstrasse 4 P.O. Box 86 07 67**
**W-8000 München 86(DE)**

(54) **Antibody-enzyme complex and enzyme immunoassay using it.**

(57) The present invention relates to an antibody-enzyme complex wherein a first antibody is conjugated with an enzyme through a bridging agent, and the enzyme is immunologically bound to a second antibody, and to an enzyme immunoassay using the antibody-enzyme complex.

EP 0 516 063 A1

The present invention relates to an antibody-enzyme complex wherein a first antibody conjugates to an enzyme through a bridging agent and the enzyme immunologically binds to a second antibody. This invention further relates to an enzyme immunoassay using the antibody-enzyme complex.

Enzyme immunoassay (EIA) is a method for detecting antigens, antibodies, or the like with the use of antibodies, antigens, or specifically reactive substance such as lectin and C1q to which enzymes, as a marker, of so high sensitivity that the activity of a very small amount of the enzyme is detectable, are chemically bound. Unlike radioimmunoassay (RIA), EIA does not have risks of radioactive contamination and does not impose restrictions on disposal of radioactive matter. Furthermore, EIA is as sensitive as RIA. Therefore, EIA is widely used now (Orita et al, Dictionary of Immunology; Saishin Igaku sha).

As a method for conjugating an antigen or an antibody with an enzyme in EIA, there are the glutaraldehyde method (Avrameas, S., Immunochemistry 6, 43-52, 1969), the periodate method (Nakane, P.K., J. Histochem. Cytochem. 22, 1084-1091, 1974), the maleimido method, the pyridil disulfide method (Carlsson, J., Biochem. J. 173, 723-737, 1987), and so on. The maleimido method and the pyridil disulfide method are beneficial in the following points; polymers are not produced, the activities of the antigen, the antibody, and the enzyme are maintained well, and the conjugating efficiency is high.

The maleimido method is based upon the fact that a maleimido group efficiently reacts with a thiol group under mild condition to form a stable bridge. That is to say, a maleimido group is introduced into an enzyme using maleimido compounds as a bridging agent, a thiol group is made on an antibody/antigen, and then both of them are reacted with each other to obtain an enzyme-conjugated antibody/antigen.

The pyridil disulfide method is based on the fact that a pyridil disulfide bond is substituted for a thiol group. That is to say, a pyridil disulfide group is introduced into an enzyme using a pyridil disulfide compound as a bridging agent, a thiol group is made on an antibody/antigen, and then both of them are reacted with each other to obtain an enzyme-conjugated antibody/antigen.

In these methods, thiol groups, which are formed by reducing disulfide bonds in a hinge region of an antibody, can be used. A method which uses thiol groups in a hinge region of Fab' fragment of an antibody is termed the hinge-enzyme-conjugating method (the hinge method). According to this method, very useful enzyme-conjugated Fab' with little nonspecific binding can be prepared without damaging the activity of an antibody (Ishikawa et al, J. Immunoassay 4 (3), 209-327, 1983). Therefore, the hinge method is usually used now.

As described above, the hinge method is the best of the enzyme-conjugating methods in EIA. In this method, however, an antibody which is naturally bivalent becomes univalent because univalent Fab' of the antibody is used for the conjugation to the enzyme, resulting in decrease of the avidity of the antibody to an antigen, especially in the case that the antigen is multivalent.

The present inventors conducted the investigation to restore the avidity of the antibody which has been decreased by the hinge method and to increase the sensitivity in EIA.

The present inventors have diligently endeavored to accomplish their purpose described above. As a result, they succeeded in remarkably restoring the avidity of the antibody by making the enzyme-conjugated antibody into a multivalent antibody-enzyme complex, that is, by binding enzyme-conjugated antibodies with another antibody against the enzyme. The antibody-enzyme complex comprises antibodies conjugated with enzymes through bridging agents and another antibody, against the enzyme, having bound to the enzymes.

Figure 1 is a graph which shows the relation of the concentration of anti-HRP McAb to the absorbance in direct ELISA which was carried out for specimens containing fixed amounts of LS 180 G50I with use of the present antibody-enzyme complex (first antibody: NKY13 antibody, bridging agent: EMCS, conjugated enzyme: HRP, and second antibody: anti-HRP McAb).

Figure 2 is a standard curve which was obtained in sandwich EIA using a bead for specimens containing fixed amounts of LS 180 G50I with use of the present antibody-enzyme complex (first antibody: NKY13 antibody, bridging agent: EMCS, conjugated enzyme: HRP, and second antibody: anti-HRP McAb).

Figure 3 is a standard curve which was obtained in sandwich EIA using a bead for hANP in which the present antibody-enzyme complex is formed during the assay (first antibody: KY-ANP-I antibody, bridging agent: EMCS, conjugated enzyme: HRP, and second antibody: anti-HRP polyclonal antibody).

This invention relates to an antibody-enzyme complex wherein a first antibody conjugates to an enzyme through a bridging agent and said enzyme immunologically binds to a second antibody. As the first antibody, any of IgG, IgM, IgA, IgD, and IgE can be used, while IgG is preferred. For example, NKY13 antibody (EP 293262) which is a monoclonal antibody against glycoprotein derived from human intestinal cancer and KY-ANP-I antibody (EP 306309) which is a monoclonal antibody against human atrial natriuretic polypeptide (hANP) can be used as the first antibody. NKY13 antibody can be prepared by the method described in EP 293262 using hybridoma NKY13" which has been deposited under the accession number 87052601 with European Collection of Animal Cell Cultures (ECACC) at PHLS CAMR, Porton Down, Salisbury, Wilts, United Kingdom since May 26, 1987 according to the Budapest Treaty. KY-ANP-I antibody can also be prepared by the method described in EP 306309 using hybridoma "KY-ANP-I" which has been deposited under the accession number 87082001 with ECACC since August 20, 1987 according to the Budapest Treaty.

Furthermore, the first antibody may be an antibody fragment capable of conjugating to the enzyme, for example, Fab, Fab', F(ab')$_2$, Fabc, and Fd , while Fab' is preferred.

As the enzyme, all enzymes usually used in EIA, for example horseradish peroxidase, $\beta$-galactosidase, glucose oxidase, alkaline phosphatase, lysozyme, glucose-6-phosphate dehydrogenase, and the like, are utilizable. Horseradish peroxidase is preferably used.

As a method for conjugating the first antibody to the enzyme, the methods using a bridging agent such as glutaric aldehyde, a maleimido compound, or a pyridil disulfide compound and the Nakaue method utilizing the oxidation by periodic acid are preferred. Especially, the method using a maleimido compound is more preferred. The maleimido compound includes N-($\epsilon$-maleimidocaproyloxy)succinimide (EMCS), N-succinimidyl-4-maleimidobutyrate, N-succinimidyl-6-maleimidohexanoate, N-succinimidyl-4-(N-maleimidomethyl)-cyclohexane-1-carbonate, and the like, while EMCS is preferred. These bridging agents are used for conjugating the first antibody to the enzyme according to conventional means.

As the second antibody, a polyclonal or a monoclonal antibody recognizing the enzyme is used. These antibodies are commercially available and can be prepared by conventional methods.

Furthermore, this invention provides an enzyme immunoassay using the antibody-enzyme complex. This enzyme immunoassay can be carried out by conventional methods such as the competitive method, the sandwich method, and the like. Selection of these methods varies according to the feature of the antigen to be determined. The avidity of the antibody-enzyme complex is much higher than that of enzyme-conjugated Fab' because the antibody valency of the complex is multivalent but that of the Fab' is univalent. Therefore, the sensitivity of this assay is increased by using the antibody-enzyme complex, as an enzyme-conjugated antibody, instead of enzyme-conjugated Fab' prepared by the hinge method.

A process of preparing this antibody-enzyme complex and an enzyme immunoassay using it are illustrated below, taking Fab' as an example of the first antibody.

1. Preparation of antibody-enzyme complex

(1) Purification of antibody

A solution of an antibody against an antigen to be determined is loaded onto a column (Protein-A Sepharose column etc.) previously equilibrated with an appropriate buffer. After washing the column, the adsorbate is eluted to obtain a pure antibody.

(2) Conjugation of enzyme to antibody

An enzyme-conjugated antibody is prepared by the method of Ishikawa et al (Ishikawa et al., J. Immunoassay 4, 209-327, 1983). The conditions for digesting the antibody with an enzyme and for reducing F(ab')$_2$ are appropriately set up subject to an used antibody.

The antibody solution purified in above (1) is applied to gel chromatography using a column equilibrated with an appropriate buffer. After the buffer exchange, the resultant is concentrated up to a desirable concentration. A pepsin solution prepared with the same buffer is added so that the ratio of the pepsin to the antibody reaches one in a few hundred by weight, followed by allowing to stand at 0 to 50°C in a water bath for dozens of minutes to several hours. The reaction is stopped by raising pH of the solution with alkali such as Tris. The resultant is loaded onto a column equilibrated with an appropriate buffer to carry out gel filtration with the same buffer. The absorbance of every several hundreds $\mu$l of the eluate is measured to calculate the amount of protein, and the main peak is collected as a F(ab')$_2$ fraction.

The obtained F(ab')$_2$ fraction is concentrated up to a desirable concentration, to which 2-mercaptoethylamine (2-MEA) prepared with phosphate buffer (PB) containing ethylendiaminetetraacetic acid (EDTA) is added enough to reduce disulfide bonds in F(ab')$_2$, followed by allowing to stand at 0 to 50°C in a water bath for dozens of minutes to several hours. The resultant is loaded onto a column equilibrated with the said PB to carry out gel filtration. The amount of protein is calculated from the absorbance of every several hundreds $\mu$l of the eluate, and the main peak is recovered as a Fab' fraction. The obtained Fab' fraction is concentrated up to a desirable concentration. The number of SH groups per Fab' molecule is counted by the method of Ishikawa et al (Ishikawa, E. et al., ibid.).

In the meantime, an appropriate bridging reagent is added to an enzyme solution which will be conjugated to an antibody, followed by agitation at 0 to 50°C for dozens of minutes to several hours. The resultant is centrifuged, and the resulting supernatant is applied to gel filtration to obtain a void fraction. The void fraction is equivalently mixed with the previously prepared Fab' fraction and allowed to stand at 0 to 50°C for several hours to dozens of hours, followed by gel filtration of the resultant. The absorbance of every several hundreds µl of the eluate is measured, and the first peak is recovered as a crude enzyme-conjugated antibody fraction. The crude fraction is purified to obtain an enzyme-conjugated antibody.

Other antibody fragments and intact antibodies are also conjugated to an enzyme by the conventional methods.

(3) Preparation of antibody-enzyme complex

The enzyme-conjugated antibody solution prepared in above (2) is reacted with an antibody against the enzyme to obtain an antibody-enzyme complex. The preparation of the complex may be carried out during an enzyme immunoassay by simultaneously adding them in the assay system at its beginning , or before the assay, the complex may have obtained by reacting them at 0 to 50°C for several minutes to several hours.

2. Enzyme immunoassay

Enzyme-linked immunosorbent assay (ELISA) using antigen-immobilized plates

(1) Preparation of antigen-immobilized plate

An antigen-immobilized plate can be prepared by the method described in JP KOKAI No. 62-212568. That is to say, an antigen solution is dispensed into each well of a microtiter plate and allowed to stand at room temperature for several hours. Then, preferably, L-polylysine is added to each well and mixed, followed by allowing to stand for at least 10 hours to obtain an antigen-immobilized plate.

(2) ELISA

The antigen-immobilised plate prepared in above (1) is washed with a blocking solution such as phosphate buffered saline (PBS) containing bovine serum albumin (BSA) and Tween 20. Then, the blocking solution is dispensed into each well, followed by incubation at room temperature for several hours to effect blocking. After the blocking solution is removed with suction, the antibody-enzyme complex solution prepared in above 1. (3) is added to each well, followed by reaction at 0 to 50°C for several hours on standing. After the reaction, each well is washed. Then, a chromogenic substrate solution such as $H_2O_2$ and ABTS {a trade name for 2,2'-Azino-bis (3-ehtylbenzthiazoline-6-sulufonic acid)} in citrate buffer (CB) is added to each well and allowed to react at room temperature for dozens of minutes to several hours, followed by addition of a stop solution such as $NaN_3$ in CB. A part of the resultant is transferred to another new polystyrene microtiter plate to measure the absorbance

In this assay, a microtiter plate on which an antigen is not immobilized is used as a blank.

Sandwich EIA using antibody-immobilized beads

(1) Preparation of antibody-immobilized beads

Beads are immersed in ethanol for about ten minutes to dozens of minutes, and then washed with an appropriate buffer such as PBS. After the same buffer is added, deairing is carried out for dozens of minutes, followed by allowing to stand at 0 to 50°C in a water bath for dozens of minutes.

The antibody solution is adjusted to an adequate concentration, deaired, and then allowed to stand at 0 to 50°C in a water bath for dozens of minutes.

The buffer in which the beads have been immersed is removed with suction, to which the antibody solution is added, followed by allowing to stand at 0 to 50°C in a water bath with stirring a few times successively at 0 to 50°C in an incubator for a few hours to several ten hours. The antibody solution is removed with suction, and the beads are washed, to which a blocking solution such as BSA in PBS is added, followed by allowing to stand at 0 to 50°C in a water bath for several hours to effect blocking. The blocking solution is removed with suction, and the beads are washed, to which the same solution is added, followed by preservation at 4°C.

(2) Sandwich EIA using beads

An antigen solution prepared with an appropriate buffer such as casein in PBS, and antibody-enzyme complex solution prepared in above 1.(3) are placed in a tube and stirred, into which one piece of the antibody-immobilized bead prepared in above (1) is put. After reaction at room temperature for at least 10 hours, the bead is washed. The bead is transferred into a new tube, to which a chromogenic substrate solution such as $H_2O_2$ and ABTS in CB is added, followed by reaction at 0 to 50°C for dozens of minutes. The reaction is terminated by addition of a stop solution such as $NaN_3$

in CB, and then the absorbance of the reaction mixture is measured at 415nm.

The present invention will be illustrated by reference to the following examples, but these examples are not intended to restrict the present invention.

Example 1

1. Materials

NKY13 antibody was produced by the method described in EP 293262 using the hybridoma "NKY13" which has been deposited under the accession number 87052601 with European Collection of Animal Cell Cultures (ECACC) at PHLS CAMR, Porton Down, Salisbury, Wilts, United Kingdom since May 26, 1987 according to the Budapest Treaty. Glycopeptide derived from LS 180 cell (LS 180 G50I), which was a standard antigen, was purchased from Bio Chiba Co., Ltd. BSA (cystal), pepsin, horseradish peroxidase (HRP), 2-mercaptoethylamine (2-MEA), $\alpha$-methylmannoside, and L-polylysine were purchased from Sigma Chemical Co., and ABTS and casein were from Boehringer Mannheim. N-( $\epsilon$ -maleimidocaproyloxy)succinimide (EMCS) was purchased from Dojin Chemical Co., and dimethyl sulfoxide (DMSO), glutaraldehyde, and Tween 20 were from Nacalai Tesque, Inc.

Protein-A Sepharose CL-4B column, Con-A Sepharose column, CNBr-activated Sepharose 4B column, and PD-10 column were purchased from Pharmacia Co. Centricon 10, 30 were from Amicon Co., and polyvinylchloride microtiter plate was from Falcon Co. Polystyrene beads (1/4 inch in diameter) were purchased from Immunochemical and Bax Co.

Anti-HRP monoclonal antibody (anti-HRP McAb) was purchased from Zymet Co., and human serum was from Miles Co.

Superose 12 column chromatography was carried out on FPLC system (pump P-500 (two), controller LCC-500, UV monitor UV-1, fraction collector FRAC-100) (Pharmacia). For measurement of the absorbance, Spectrophotometer UV-265 (Shimadzu Corporation) and Immuno reader NJ-2000 (Intermed) were used.

2. Preparation of an antibody-enzyme complex

(1) Purification of NKY13 antibody

One hundred mg/17.5ml NKY13 antibody (Lot. BCS-1), which was prepared by equivalently mixing 0.01M borate buffered saline (BBS) (pH 8.5) with BCS-1 to adjust pH of the mixture to 8.5, was loaded onto Protein-A Sepharose CL-4B column (1.5cm × 10cm) previously equilibrated with 0.01M BBS (pH 8.5). After the column wag washed with 0.01M BBS (pH 8.5) and 0.01M PBS (pH 6.0), the adsorbate was eluted with 0.01M citrate buffered saline (CBS) (pH 4.0). The pH value of the eluate was raised immediately after the elution by use of the fraction tubes already charged with 1M Tris-HCl (pH 8.5). The fraction obtained by the elution (pH 4.0) was collected as a pure NKY13 antibody.

(2) Preparation of an enzyme-conjugated antibody

An enzyme-conjugated antibody was prepared according to the method of Ishikawa et al (Ishikawa, E. et al., J. Immunoassay 4, 209-327, 1983). The conditions for digesting the antibody with an enzyme and for reducing F(ab')$_2$ were newly set up on NKY13 antibody.

The NKY13 antibody solution, which was purified by Protein-A in above (1), was applied to gel chromatography using PD-10 column previously equilibrated with 0.1M CB (pH 4.8) to carry out buffer exchange, and then concentrated up to 5.5mg/ml by means of Centricon 30. The pepsin solution, which was prepared to be 1mg/ml with 0.1M CB (pH 4.8), was added so that the ratio of the pepsin / the antibody reached 1/400 by weight, followed by reaction at 37°C in a water bath for 30 minutes. The reaction was stopped by raising pH of the solution with 1M Tris. The resultant was loaded onto a Superose 12 column (16/50) previously equilibrated with 0.1M PB (pH 6.0) containing 5mM EDTA, and eluted with the same buffer at a flow rate of 1.5ml/min. The absorbance of every 0.75ml of the eluate was measured at 280nm to calculate the amount of protein, and a main peak was collected as a F(ab')$_2$ fraction.

The obtained F(ab')$_2$ fraction was concentrated up to 5mg/ml by means of Centricon 30, to which 1/9 volumes of 25mM 2-MEA prepared with 0.1M PB (pH 6.0) containing 5mM EDTA was added (final concentration of 2-MBA was 2.5mM.), followed by allowing to stand at 25°C in a water bath for 60 minutes. The resultant was loaded onto a Superose 12 column (16/15) previously equilibrated with 0.1M PB (pH 6.0) containing 5mM EDTA, and eluted at a flow rate of 1.5ml/min. The amount of protein was calculated from the absorbance of every 0.75ml of the eluate at 280nm, and a main peak was collected as a Fab' fraction. The obtained Fab' fraction was concentrated up to 4.0mg/ml by means of Centricon 10. The number of SH groups per Fab' molecule was counted by the method of Ishikawa et al (Ishikawa, E. et al., ibid).

In the meantime, 11mg/1.65ml HRP prepared with 0.1M PB (pH 7.0) was reacted with 8.8mg of EMCS in 110 $\mu$l of DMSO at 0°C for 1 hours with agitation. The resultant was centrifuged at 3,000rpm for 10 minutes. The resulting supernatant was loaded onto PD-10 column previously equilibrated with 0.1M PB (pH 6.0), and the void fraction was collected as maleimidated HRP. The obtained maleimidated HRP, whose concentration was adjusted to 3.6mg/ml, was equivalently mixed with the Fab' fraction prepared in the above, followed by allowing to stand at 4°C for 20 hours. The resultant was loaded on to Superose 12 (16/50) previously equilibrated with 0.05M Tris-HCl (pH 7.2) containing 0.2M NaCl, and eluted with the same buffer at a flow rate of 1.5ml/min. The absorbance of every 0.75ml of the eluate was measured at 280nm, and the first peak was collected as a crude enzyme-conjugated antibody fraction.

CaCl$_2$ and MnCl$_2$ were added to the crude enzyme-conjugated antibody fraction so that each concentration reached 1mM. The fraction was loaded onto Con A Sepharose column (1.0 × 1.5cm) previously equilibrated with 0.05M Tris-HCl (pH 7.2) containing 0.2M NaCl, 1mM CaCl$_2$, and 1mM MnCl$_2$. The column was washed with the same buffer. The adsorbate was eluted with the same buffer containing 0.5M α-methylmannoside and collected as a pure enzyme-conjugated antibody.

(3) Preparation of antibody-enzyme complex solution

A solution containing a final concentration 10 μg/ml of the enzyme-conjugated antibody prepared in above (2) and a final concentration 1, 3, 10, or 30 μg/ml of anti- HRP McAb prepared with PBS containing 0.1% casein was allowed to stand at room temperature for 2 hours to obtain an antibody-enzyme complex solution.

As a negative control, 10 μg/ml enzyme-conjugated antibody of above (2) prepared with 0. 1% casein in PBS was used.

3. Increase of sensitivity in sandwich EIA

Direct ELISA using antigen-immobilized plate

(1) Preparation of antigen-immobilized plate

Twenty μl of the mixture of 100 μg/100ml LS 180 G50l, 1.9ml of 0.01M PBS (pH 7.4), and 30 μl of 0.25% glutaraldehyde in PBS was dispensed into each well of a polyvinyl chloride microtiter plate, and allowed to stand at room temperature for 1 hour. Then, 10 μl of L-polylysine, the concentration of which was adjusted to 0.25mg/ml with 0.01M PBS, was added to each well, followed by allowing to stand at 4°C for at least 18 hours to obtain an antigen-immobilized plate.

(2) ELISA

Each well of the antigen-immobilized plate prepared in above (1) was washed 3 times with 200 μl of 0.01M PBS (pH 7.4) containing 1% BSA and 0.05% Tween 20 (hereinafter referred to as Tween buffer). Then, 200 μl of Tween buffer was dispensed into each well, followed by incubation at room temperature for 1 hour to effect blocking. After the buffer was removed with suction, 20 μl of the antibody-enzyme complex solution or the negative control solution, which were prepared in above 2. (3), was dispensed into each well. The plate was allowed to stand at 25°C for 4 hours to react the antibody-enzyme complex with the antigen. Each well was washed 3 times with 200 μl of Tween buffer. Then, 100 μl of a chromogenic substrate solution consisting of 2mM H$_2$O$_2$ and 4.3mM ABTS in 0.1M CB (pH 4.5) was dispensed into each well. After reaction at room temperature for 30 minutes, 100 μl of 0.76mM NaN$_3$ in 0.1M CB (pH 4.5) was added to terminate the reaction. Then, 150 μl of the resultant was transferred to another new polystylen microtiter plate, and the absorbance was measured at 415nm or 450nm when the value of the absorbance is high.

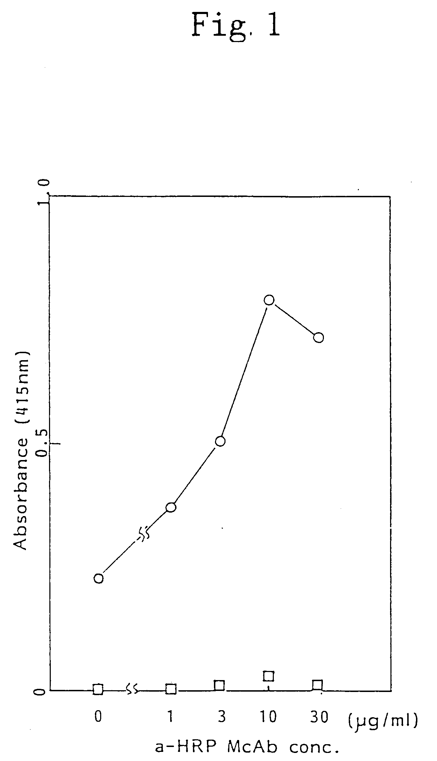

As a result, the absorbance increased in proportion to the increase of the concentration of anti-HRP McAb in the antibody-enzyme complex solution, and when the concentration of anti-HRP McAb is 10 μg/ml, the reactivity increased about 4 times as much as that in the absence of anti-HRP McAb (-○-in Figure 1).

In this assay, an microtiter plate on which no antigen was immobilized was used as a blank (-□-in Figure 1).

Sandwich EIA using beads

(1) Preparation of NKY13 antibody-immobilized beads

Polystyrene beads (1/4 inch in diameter) were immersed in 0.15ml of ethanol per bead for 10 minutes, and then washed 3 times with 0.2ml of 10mM PBS (pH 7.4) per bead. After 0.2ml of PBS per bead was added, deairing was carried out for 30 minutes, followed by allowing to stand at 37°C in a water bath for 30 minutes.

The concentration of the antibody solution was adjusted to 20 μg/ml with 10mM PBS (pH 7.4), deairing was carried out, and the solution was allowed to stand at 37°c in a water bath for 30 minutes.

The PBS in which the beads had been immersed was removed with suction, to which 0.2ml of the antibody solution per bead was added and mixed. The mixture was allowed to stand at 37°C in a water bath with 4 times stirring at an interval of 15 minutes, followed by transferring into a 37°C incubator and then allow to stand for 19.5 hours. After the antibody solution was removed with suction, the beads were washed 3 times with 10mM PBS (pH 7.4), to which 0.2ml of a blocking solution consisting of 0.1% BSA in PBS per bead was added, followed by allowing to stand at 37°C in a water bath for 2 hours to effect blocking. The blocking solution was removed with suction, and the beads were washed 5 times with 0.05% Tween 20 in PBS and successively 3 times with PBS, to which 0.2ml of 0.1% BSA in PBS per bead was added, followed by preservation at 4°C The beads were used in sandwich EIA after the preservation for at least 1 day.

(2) Preparation of antigen-free human serum

A mixture, which consisted of 50mg of NKY13 antibody in 20ml of 0.1M bicarbonate buffer (pH 8.3) and 10ml of CNBr-activated Sepharose 4B swollen with 1mM HCl, was reacted at room temperature for 2 hours under stirring, and filtered through a glass filter to collect the Sepharose gel. The gel was blocked with 1M ethanolamine (pH 8.0) to obtain NKY13 antibody-immobilized Sepharose 4B.

Thirty ml of human serum was loaded at a flow rate of 0.3ml/min. onto the NKY13 antibody-immobilized Sepharose 4B column (1.0 × 8.0cm) previously equilibrated with 0.01M PBS (pH 7.4). The first 10ml of the eluate was removed, and the rest was recycled to adsorb antigens existing in the human serum.

(3) Preparation of standard solution

LS 180 G50I was adjusted to 0.078, 0.313, 1.25, 5, and 10 μg/ml with PBS containing 0.1% casein or with the antigen-free human serum prepared in above to give standard solutions.

(4) Sandwich EIA using beads

Twenty μl of the standard solution prepared in above (3), 100 μl of PBS containing 0.1% casein, and 50 μl of the antibody-enzyme complex solution containing 10 μl/ml anti-HRP McAb prepared in above 2. (3) were placed and stirred in a tube, into which one piece of the NKY13 antibody-immobilized bead was put. After the reaction at room temperature for 18 hours, the bead was washed 3 times with 2ml of 0.05% Tween 20 in PBS. The bead was transferred into another new tube, to which 300 μl of a chromogenic substrate solution consisting of 2mM $H_2O_2$ and 4.3mM ABTS in 0.1M CB (pH 4.5) was added, followed by reaction at 37°C for 30 minutes. The reaction was terminated by addition of 2ml of 0.38mM $NaN_3$ in 0.1M CB (pH 4.5). The absorbance of the resultant was measured at 415nm.

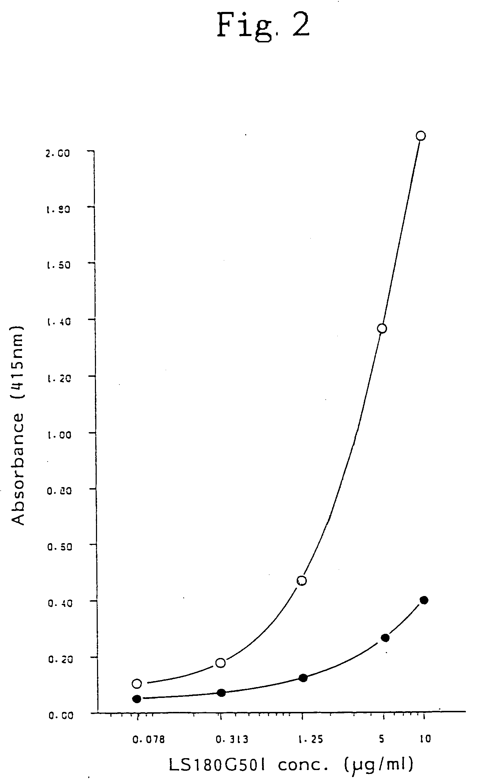

At the same time, using the negative control solution of an antibody-enzyme complex solution, the above process was carried out. As a result, the presence of anti-HRP McAb (-O- in Figure 2) increased the reactivity and the sensitivity 4 times as comparison to those in the absence (-●- in Figure 2).

Furthermore, by changing 0.1% casein-containing PBS into an antigen-free human serum as a solvent of the standard solution, the absorbance of the blanks at 415nm could be decreased from 0.08 - 0.09 to 0.028.

Example 2

1. Preparation of enzyme-conjugated antibody

Using KY-ANP-I which is a monoclonal antibody against hANP, an enzyme-conjugated KY-ANP-I was prepared according to the method of Ishikawa et al (Ishikawa, E. et al., ibid.) in the same manner as Example 1.

KY-ANP-I antibody can be prepared by the method described in EP 306309 using hybridoma "KY-ANP-I" which has been deposited under the accession number 87082001 with European Collection of Animal Cell Cultures (ECACC) at PHLS CAMR, Porton Down, Salisbury, Wilts, United Kingdom since August 20, 1987 according to the Budapest Treaty.

The obtained enzyme-conjugated antibody was dissolved in phosphate buffer containing 0.1% BSA so that the concentration reached 500mg/ml, and then used in EIA.

2. Preparation of anti-HRP antibody solution

An anti-HRP polyclonal antibody (Dako Co.) was dissolved in phosphate buffer containing 0.1% BSA so that the concentration reached 0, 100, or 200ng titer/ml, and then used in ETA.

3. Preparation of AN111 antibody-immobilized beads

AN111 antibody-immobilized beads, of which antibody is a monoclonal antibody recognizing C-terminus of ANP, were prepared according to the same method as that for NKY13 antibody-immobilized bead in Example 1.

The AN111 antibody was prepared by the method described in EP 393640 using "Mouse hybridoma AN111" which has been deposited under the accession number FERM BP-2197 with Fermentation Research Institute, Agency of Industrial Science and Technology at 1-3, Higashi 1 chome Tsukuba-shi Ibaraki-ken 305, Japan since December 20, 1988 according to the Budapest Treaty.

4. Preparation of standard solution

hANP (Peptide Institute, Inc.) was dissolved in phosphate buffer containing 0.1% BSA so that the concentration reached 20, 60, 200, 600, or 2000 pg/ml, and then used in EIA.

5. Sandwich EIA using beads

One hundred $\mu$l of the standard solution, 100 $\mu$l of the enzyme-conjugated antibody solution, and 100 $\mu$l of anti-HRP antibody solution were placed and stirred in a tube, into which one piece of the AN111 antibody-immobilized bead was put. After the reaction at 4°C for 20 hours, the bead was washed 3 times with 2ml of 0.05% Tween 20 in PBS. The bead was transferred into another new tube, to which 300 $\mu$l of a chromogenic substrate solution (0.1M CB, pH 4.5, containing of 2mM $H_2O_2$ and 4.3mM ABTS) was added, followed by reaction at 37°C for 30 minutes. The reaction was terminated by addition of 2ml of 0.1M CB (pH 4.5) containing 0.38mM $NaN_3$. The absorbance of the resultant was measured at 415nm.

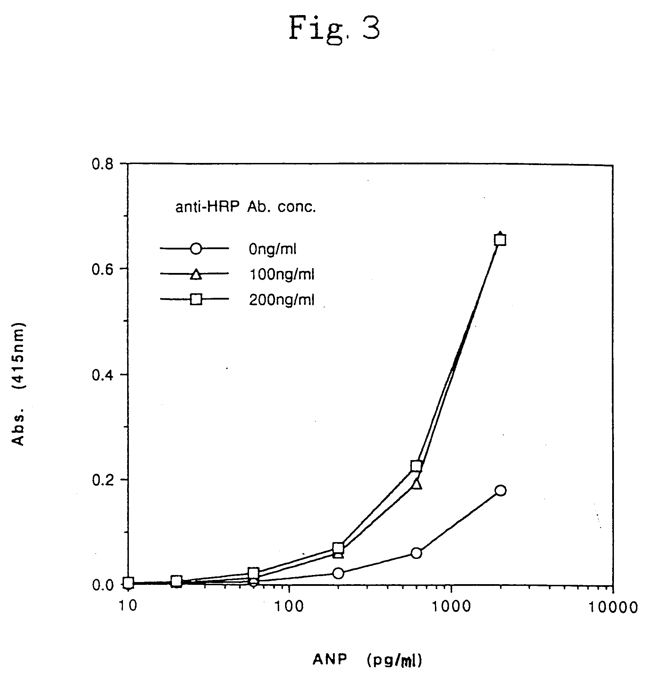

As a result, the presence of 100ng/ml and 200ng/ml anti-HRP antibody (respectively, -△- and -□- in Figure 3) increased the reactivity and the absorbance 3 times as much as those in the absence (-○- in Figure 3).

## Claims

1. An antibody-enzyme complex, wherein a first antibody conjugates to an enzyme through a bridging agent and said enzyme immunologically binds to a second antibody.

2. The complex of Claim 1, wherein said first antibody is an antibody fragment.

3. The complex of Claim 2, wherein said antibody fragment is Fab'.

4. The complex of Claim 1, wherein said first antibody is IgG.

5. The complex of Claim 1, wherein said first antibody is NKY13 antibody or KY-ANP-I antibody.

6. The complex of any one of Claims 1 to 5, wherein said enzyme is horseradish peroxidase.

7. The complex of any one of Claims 1 to 6, wherein said bridging agent is N-($\epsilon$-maleimidocaproyloxy)succinimide.

8. An enzyme immunoassay using the complex of any one of Claims 1 to 7.

9. A kit comprising the complex of any one of Claims 1 to 7.

# Fig. 1

# Fig. 2

# Fig. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-9 007 714 (APPLIED RESEARCH SYSTEMS ARS HOLDING NV)<br>* abstract * | 1,8,9 | G01N33/535<br>G01N33/58 |
| A | US-A-4 578 350 (R.D.ARMENTA ET AL.)<br>* column 3 - column 5 * | 1,8,9 | |
| A,D | EP-A-0 306 309 (SHIONOGI SEIYAKU KABUSHIKI KAISHA)<br>* the whole document * | 1-7 | |
| A,D | EP-A-0 293 262 (I.YAMASHINA)<br>* claims * | 5 | |
| E | EP-A-0 487 301 (SYNTEX (U.S.A) INC.)<br><br>* the whole document * | 1-3,6,8,9 | |

TECHNICAL FIELDS SEARCHED (Int. Cl.5)

G01N

The present search report has been drawn up for all claims

| Place of search | Date of completion of the search | Examiner |
|---|---|---|
| THE HAGUE | 17 AUGUST 1992 | HITCHEN C.E. |

EPO FORM 1503 03.82 (P0401)